(19) Europäisches Patentamt
European Patent Office
Office européen des brevets

(11) Publication number: 0 028 459 A2

(12) **EUROPEAN PATENT APPLICATION**

(21) Application number: 80303474.3

(22) Date of filing: 02.10.80

(51) Int. Cl.$^3$: C 07 C 69/533
A 61 K 7/00, C 11 D 1/74
//C07C67/26

(30) Priority: 31.10.79 GB 7937696

(43) Date of publication of application: 13.05.81 Bulletin 81/19

(84) Designated Contracting States: AT BE CH DE FR GB IT LI LU NL SE

(71) Applicant: IMPERIAL CHEMICAL INDUSTRIES LIMITED
Imperial Chemical House Millbank
London SW1P 3JF(GB)

(72) Inventor: Ludwig, Klaus-Günther
am Ruhrstein 45
D-4300 Essen(DE)

(74) Representative: Jones, Martin et al,
Imperial Chemical Industries Limited Legal Department: Patents Thames House North Millbank
London SW1P 4QG(GB)

(54) **Surfactants derived from undecylenic acid, and cosmetic compositions containing them.**

(57) Alkoxylated polyhydric alcohol esters of undecylenic acid, especially sorbitol and anhydro-sorbitol based esters, which are surfactants and may be used in cosmetics, including hair shampoos.

EP 0 028 459 A2

Croydon Printing Company Ltd.

TITLE MODIFIED
see front page

0028459

31025 EP



## SURFACTANTS DERIVED FROM UNDECYLENIC ACID, THEIR PREPARATION AND USES

This invention relates to surfactants containing within the molecule at least one group derived from undecylenic acid. The surfactants are nonionic, having a lipophilic component which is the undecylenic group itself, and hydrophilic groups which are a polyoxyalkylene chain, and perhaps hydroxy groups.

## BACKGROUND REFERENCES

Polyethylene glycol mono-esters of fatty acids including undecylenic acid are known (Rao et al., Fats Oils Relat. Food Products Symposium 1976 published 1978 by Association of Food Service and Technology of India, Mysore, India).

The methyl ether derivative of ethoxylated undecylenic acid is known from French Patent 1 522 750.

The mono-ethanolamide derivative of undecylenic acid has been ethoxylated to produce a fungicidal preparation (British Patent No. 981 416). Ester derivatives of such compounds with for example succinic acid are also known (British Patent No. 1 018 302).

Undecylenic acid and its derivatives find use in cosmetic compositions, especially shampoos. While their precise activity against bacteria and fungi is not clearly established, undecylenic acid based compositions are ofter used where mild non-specific bactericidal or fungicidal activity is sought. In the present invention, the undecylenic acid is contained in a compound which has surfactant and hence cleaning properties and is readily miscible with water.

## THE INVENTION

The present invention provides surfactants which are alkoxylated polyhydric alcohol esters of undecylenic acid, containing 1 to n residues of undecylenic acid, the polyhydric alcohol containing from 3 to 7 carbon atoms and, originally, n OH-groups and where n is an integer and is at least 2.

The invention also provides cosmetic compositions including shampoos containing surfactants of the type defined above.

The surfactants may also be described as compounds of the general formula :-

$$\left[R - \overset{O}{\overset{\|}{C}} — O \left( R' — O \right)_b \right]_c R'' \left[ \left( O — R' \right)_b OH \right]_{n-c}$$

where R = undecylenic acid residue

R' = a $C_2$ alkylene residue, optionally with substituents such as $-CH_3$

R'' is a hydrocarbon residue of polyhydric alcohol containing from 3 to 7 carbon atoms

b is at least 1, preferably from 1 to 100 and may be different in different parts of the molecule

c is from 1 to a maximum of n where n is the number of hydroxyl groups in the polyhydric alcohol referred to in the definition of R'' above, n being an integer and at least 2.

Examples of Groups R'' are as the following

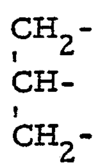
n = 3

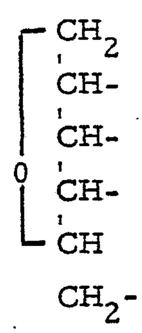
n = 4

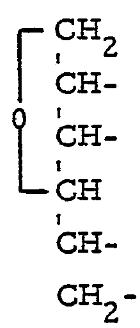
n = 4

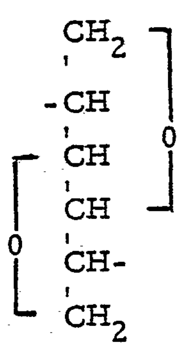
n = 2

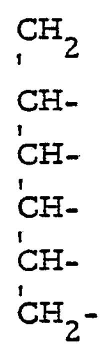
n = 6

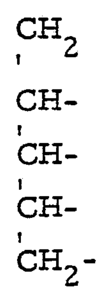
n = 5

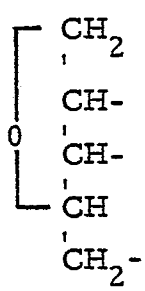
n = 3

n is preferably from 2 to 6.

Examples of compounds falling within the scope of the invention include :-

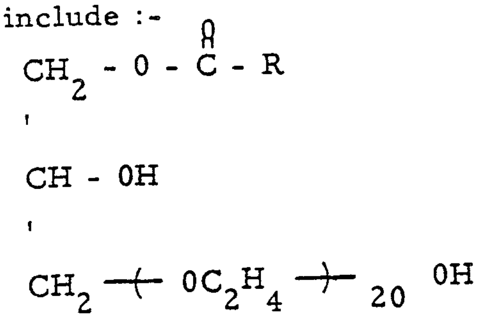

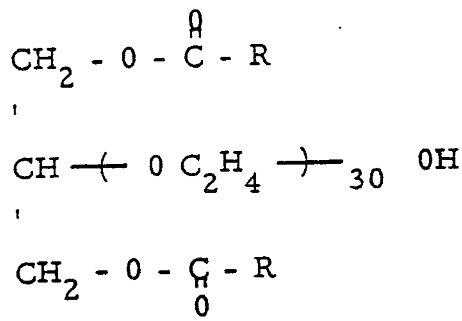

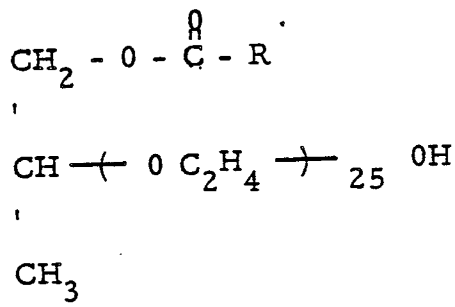

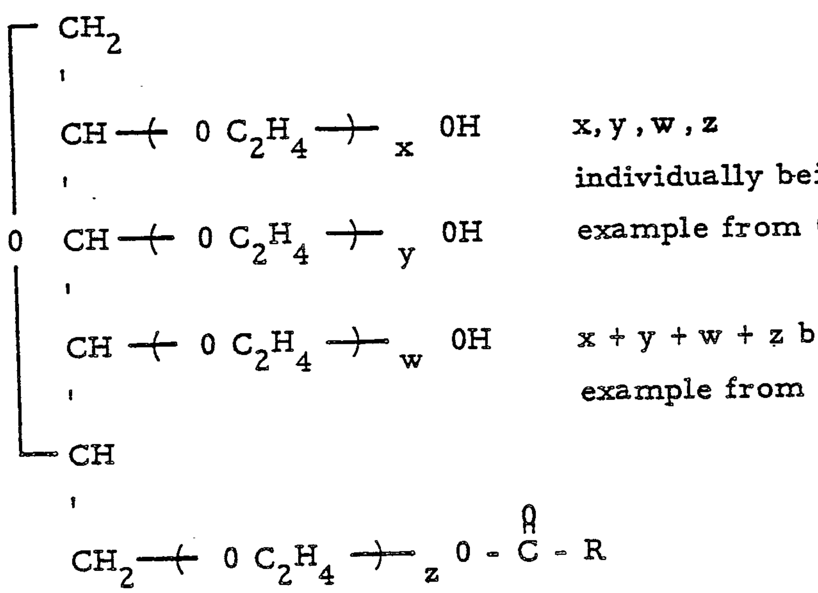

x, y, w, z individually being, for example from 0 to 20 and

x + y + w + z being, for example from 10 to 40

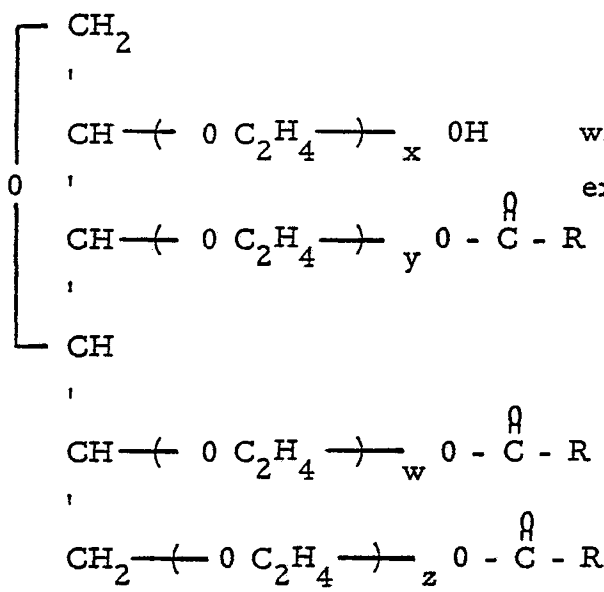

where x, y, w, z have, for example, the values specificed above.

The compounds can be prepared according to known methods for example the acid R - C00H can be

(a) esterified with a polyalkoxy ether of a polyhydric alcohol and optionally further alkoxylated.

or

(b) esterified with a polyhydric alcohol and subsequently alkoxylated.

Alternatively a polyhydric alcohol can be first alkoxylated and the reaction product esterified with undecylenic acid.

The compounds of the invention are surfactants and are useful in the preparation of skin- and hair-care products. They are particularly suitable for incorporation into shampoos.

Preferred compounds for this purpose include :-

(a) The alkoxylation products obtained by reacting one mole of undecylenic and with 2 to 20 moles ethylene oxide.

(b) The products obtained by reacting one mole of undecylenic acid with one mole of a $C_3$ - $C_6$ polyhydric alcohol (like glycerol, 1,2-propylene glycol, sorbitol, 1,4-sorbitan, or 1,5-sorbitan) and by subsequent alkoxylation with 5 to 40 moles ethylene oxide.

(c) The products obtained by reacting 2 to n moles of undecylenic acid with a $C_3$ - $C_6$ polyhydric alcohol containing n OH groups and by subsequent alkoxylation with 5 to 60 moles ethylene oxide.

Incorporated into shampoo formulations at about 5-20% by weight of the total formulation, surfactants according to the invention have been shown to reduce the incidence of dandruff on some test subjects.

The invention is illustrated by the following Examples.

Example 1

Sorbitan monoundecylate (7, 96 kg) and sodium hydroxide (50 g solid) were charged to a reactor equipped with heater and stirrer. Ethylene oxide was supplied under pressure (max. 3, 5 bar) and the reactor heated to 180 ± 5°C, with stirring. The amount of ethylene oxide supplied (22. 04 kg) was calculated to give 20 mols of ethoxy units per mol undecylenic. The product surfactant was recovered by vacuum treatment at 120°C after which 0,5% weight hydrogen per oxide was introduced at 90°C. After further vacuum treatment at 120°C, phosphoric acid (10% in weight in water) was added to adjust to pH 6-8. The product was filtered at 90°C and the water content adjusted to 2. 5°-3. 0% by weight at 70°C. The product was trasferred to containers.

Example 2

Ethoxylated sorbitantriundecylenate was prepared by the procedure of Example 1 using the following quantities and conditions. The product had twenty ethoxy units per mole of sorbitan triundecylenate

| | |
|---|---|
| Sorbitantriundecylenate | 5 Kg |
| Sodium hydroxide | 23 Kg |
| Ethylene oxide | 6, 73 Kg |
| Reaction temperature | 160 ± 5°C |
| Maximum reaction pressure | 3, 5 bar |

SURFACTANTS DERIVED FROM UNDECYLENIC ACID, THEIR PREPARATION AND USES

CLAIMS

1. Alkoxylated polyhydric alcohol esters of undecylenic acid containing 1 to n residues of undecylenic acid, the polyhydric alcohol containing from 3 to 7 carbon atoms and originally n hydroxyl groups, where n is an integer and is at least 2.

2. A compound of the general formula

$$\left[ R - \overset{O}{\overset{\|}{C}} - O \left( R' - O \right)_b \right]_c R'' \left[ \left( O - R' \right)_b OH \right]_{n-c}$$

where R = undecylenic acid residue

R'= a $C_2$ alkylene residue, optionally with substituents such as $-CH_3$

R'' is a hydrocarbon residue of polyhydric alcohol containing from 3 to 7 carbon atoms

b is at least 1, preferably from 1 to 100 and may be different in different parts of the molecule

c is from 1 to a maximum of n where n is the number of hydroxyl groups in the polyhydric alcohol referred to in the definition of R'' above, n being an integer and at least 2.

3. A compound according to claim 1 or claim 2 in which n is from 2 to 6.

4. Cosmetic compositions, including shampoos, containing as an ingredient, a compound as claimed in any one of claims 1 to 3.